# DEMANDE DE BREVET EUROPEEN

(11) **EP 1 245 223 A1**
(43) Date de publication de la demande: **02.10.2002**
(21) Numéro de dépôt: 02290722.4
(22) Date de dépôt: 21.03.2002
(51) Int. Cl.: A61K 7/48

(54) **Composition cosmétique comprenant un mélange de fibres**

(30) Priorité: 30.03.2001 FR 0104392
(71) Demandeur: L'OREAL, 75008 Paris (FR)
(72) Inventeur: Collin, Nathalie, 92330 Sceaux (FR)
(74) Mandataire: Kromer, Christophe

(57) **Abrégé**

L'invention a pour objet une composition comprenant, dans un milieu aqueux physiologiquement acceptable, une première fibre choisie parmi les fibrilles de cellulose et une deuxième fibre différente de ladite première fibre.

La composition permet d'obtenir un bon maintien des fibres sur les matières kératiniques.

Application au maquillage et au soin des matières kératiniques, en particulier en mascara.

## Description

La présente invention se rapporte à une composition comprenant, dans un milieu aqueux physiologiquement acceptable, un mélange de fibres, destinée en particulier au domaine cosmétique. L'invention se rapporte également à un procédé de maquillage ou de soin cosmétique des matières kératiniques. La composition et le procédé de maquillage ou de soin selon l'invention sont plus particulièrement destinés aux matières kératiniques d'êtres humains telles que la peau (y compris les lèvres), les ongles, les fibres kératiniques, notamment sensiblement longitudinales, telles que les cils, les sourcils et les cheveux. Plus spécialement, l'invention porte sur un mascara.

La composition selon l'invention peut être une composition de maquillage ou de soin des matières kératiniques, notamment se présenter sous la forme de composition de revêtement des cils (notamment de mascara), d'eye-liner, de produit pour les sourcils, de produit pour les lèvres, de fard à joues ou à paupières, de fond de teint, de produit de maquillage du corps, de produit anti-cernes, de vernis à ongles, de produit de soin de la peau, y compris du cuir chevelu, de produit pour les cheveux (mascara pour cheveux).

Il est connu d'utiliser des fibres dans des produits de maquillage notamment pour leurs effets allongeants dans des mascaras (voir JP-A-57/158714, JP-A-3-153613), leurs propriétés hydratantes dans des rouges à lèvres (voir le document US-A-5 498407), pour améliorer les contours du rouge à lèvres sur les bords des lèvres (voir le document EP-A-0106762) ou pour remettre en état les ongles cassés (voir FR-A-1529329) ou bien encore dans des produits de soin de la peau pour leur toucher velouté (voir JP-A-7/196440).

Toutefois, après l'application de ces compositions sur les matières kératiniques, les fibres n'adhèrent pas bien sur les matières kératiniques : elles se détachent et s'éliminent des matières kératiniques dans le temps. L'élimination de ces fibres provoque alors une diminution perceptible des propriétés cosmétiques recherchées apportées par les fibres (par exemple, perte de l'effet allongeant du mascara), nécessitant de renouveler l'application du produit. De plus, pour un mascara, les fibres en se détachant des cils tombent sur les paupières et les joues conférant un aspect inesthétique ; les fibres détachées peuvent aussi se loger dans les yeux et provoquer un inconfort occulaire ou des problèmes d'intolérance occulaire.

Le but de la présente invention est de disposer d'une composition cosmétique permettant de remédier aux inconvénients mentionnés ci-dessus, comprenant des fibres adhérant bien sur les matières kératiniques.

La demanderesse a maintenant constaté de façon surprenante que l'utilisation de fibrilles de celulose dans une composition comprenant des fibres, différentes desdites microfibrilles de cellulose, permettait d'obtenir un bon maintien des fibres déposées sur les matières kératiniques. Les fibres adhèrent bien sur les matières kératiniques et ne s'en détachent pas au cours du temps. Ainsi, les propriétés cosmétiques de la composition, et notamment du maquillage, sont bien conservées au cours du temps. En particulier, lorsque la composition est un mascara, on constate que l'effet allongeant du mascara est durable dans le temps, dû au bon maintien des fibres sur les cils. Les fibres tiennent bien sur les cils et ne tombent pas sur les paupières ou les joues, ni dans les yeux, ce qui évite les problèmes d'inesthétisme ou de gêne occulaire.

De façon plus précise, l'invention a pour objet une composition comprenant, dans un milieu aqueux physiologiquement acceptable, une première fibre choisie parmi les fibrilles de cellulose et une deuxième fibre différente de ladite première fibre.

L'invention a également pour objet un procédé cosmétique de maquillage ou de soin des matières kératiniques des êtres humains comprenant l'application sur les matières kératiniques d'une composition telle que définie précédemment.

L'invention a encore pour objet l'utilisation d'une composition telle que définie précédemment pour obtenir une bonne tenue des fibres sur les matières kératiniques.

L'invention a aussi pour objet l'utilisation de fibrilles de cellulose dans une composition, notamment un mascara, comprenant dans un milieu aqueux cosmétiquement acceptable, des fibres, différentes desdites microfibrilles de cellulose, pour obtenir un bonne tenue des fibres sur les matières kératiniques, notamment les cils.

L'invention a pour autre objet l'utilisation d'un mascara comprenant une composition telle que définie précédemment pour allonger les cils et/ou obtenir une bonne tenue des fibres sur les cils.

La composition selon l'invention comprend un milieu physiologiquement acceptable contenant une phase aqueuse. Par milieu physiologiquement acceptable, on entend un milieu non toxique et susceptible d'être appliquée sur la peau, les phanères ou les lèvres d'êtres humains, comme un milieu cosmétique.

La composititon selon l'invention comprend une première fibre choisie parmi les fibrilles de cellulose.
On entend dans la présente demande par « fibrilles de cellulose » aussi bien des nanofibrilles que des microfibrilles dont la plus grande longueur est inférieure à 100 µm, et de préférence inférieure à 50 µm. Ces fibrilles ont généralement une plus grande longueur supérieure à 1 µm et de préférence allant de 5 à 40 µm. En outre, les fibrilles de cellulose peuvent avoir un diamètre allant par exemple de 1 à 100 nm (0,001 à 0,1 µm) ; le rapport plus grande longueur/diamètre peut être égal ou supérieur à 30.

Les fibrilles de cellulose utilisées selon l'invention sont de préférence amorphes, c'est-à-dire qu'elles présentent de préférence un taux de cristallinité inférieur ou égal à 50 %, et de préférence allant de 15 à 50 %.

En outre, les fibrilles de cellulose utilisées selon l'invention peuvent être obtenues soit par extraction mécanique ou chimique à partir de végétaux ou d'algues, soit par fermentation bactérienne. Par ailleurs, elles peuvent se présenter sous forme de matière sèche ou en dispersion notamment aqueuse.

Les fibrilles de cellulose utilisées selon l'invention peuvent se présenter telles quelles ou modifiées. Ainsi, elles peuvent comprendre (ou être en mélange avec) un additif et notamment avec de la cellulose, comme décrit dans les documents WO-A-98/02486 et WO-A-98/02487. Elles peuvent être également sous forme modifiée, et par exemple elles peuvent être modifiées par des acides carboxyliques comme décrit par exemple dans le document EP-A-726356, et/ou être associées à un composé organique polyhydroxylé comme décrit par exemple dans le document FR-A-2,769,836.

On peut notamment utiliser comme fibrilles de cellulose celles commercialisées sous les dénominations « cellulon » par la société Kelco, celle commercialisée sous la dénomination « Fibriliance » par la société Soliance et celles de la société Rhodia, notamment celle appelée ci-après « composé A », comprenant 85 % de nanofibrilles et 15 % de carboxyméthylcellulose et obtenues selon l'exemple 1 de la demande FR-2,769,836.

Les fibrilles de cellulose sont généralement introduites dans la phase aqueuse de la composition.

La composition de l'invention peut contenir une quantité de fibrilles de cellulose, allant de 0,01 à 5 % de matière active en poids, de préférence de 0,1 à 3 % et mieux de 0,5 à 2 % en poids de matière active, par rapport au poids total de la composition.

La composition contient également une deuxième fibre dite fibre additionnelle, différente de la première fibre décrite précédemment.
Par "fibre", il faut comprendre un objet de longueur (ou plus grande longueur) L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2 500, de préférence de 5 à 500 et mieux de 5 à 150.

Les deuxièmes fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les deuxièmes fibres ont une longueur (ou plus grande longueur) supérieure ou égale à 100 µm, notamment allant de 100 µm à 10 mm, de préférence allant de 0,1 mm à 5 mm et mieux allant de 1 mm à 3,5 mm. Leur section peut être comprise dans un cercle de diamètre allant de 2 nm à 500 µm, de préférence allant de 100 nm à 100 µm et mieux de 1 µm à 50 µm. Le poids ou titre des fibres est souvent donné en denier ou décitex et représente le poids en gramme pour 9 km de fil. De préférence, les deuxièmes fibres selon l'invention ont un titre choisi dans la gamme allant de 0,15 à 30 deniers et mieux de 0,18 à 18 deniers.

Les deuxièmes fibres peuvent être celles utilisées dans la fabrication des textiles et notamment des fibres de soie, de coton, de laine, de lin, des fibres de cellulose - notamment extraites notamment du bois, des légumes ou des algues -, de rayonne, de polyamide (Nylon ®), de viscose, d'acétate notamment d'acétate de rayonne, de poly-(p-phénylène-téréphtalamide) (ou d'aramide) notamment de Kevlar ®, de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène (comme le Téflon ®), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.

On peut aussi utiliser les fibres utilisées en chirurgie comme les fibres synthétiques résorbables préparées à partir d'acide glycolique et ε-caprolactone (Monocryl de chez Johnson & Johnson) ; les fibres synthétiques résorbables du type copolymère d'acide lactique et d'acide glycolique (Vicryl de chez Johnson & Johnson) ; les fibres de polyester téréphtalique (Ethibond de chez Johnson & Johnson) et les fils d'acier inoxydable (Acier de chez Johnson & Johnson) notamment pour une application en vernis à ongles.

Par ailleurs, les deuxièmes fibres peuvent être traités ou non en surface, enrobées ou non. Comme fibres enrobées utilisables dans l'invention, on peut citer des fibres de polyamides enrobées de sulfure de cuivre pour un effet anti-statique (par exemple le R-STAT de chez Rhodia) ou un autre polymère permettant une organisation particulière des fibres (traitement de surface spécifique) ou traitement de surface induisant des effets de couleurs/hologrammes (fibre Lurex de chez Sildorex, par exemple).

De préférence, on utilise des fibres d'origine synthétiques et en particulier des fibres organiques, comme celles utilisées en chirurgie. Avantageusement, on peut utiliser des fibres insolubles dans l'eau.

Les deuxièmes fibres utilisables dans la composition selon l'invention sont préférentiellement des fibres de polyamide, de cellulose, de poly-phénylène téréphtalamide ou de polyéthylène. Leur longueur (L) peut aller de 0.1 à 5 mm, de préférence de 0.25 à 3 mm et leur diamètre moyen peut aller de 1 à 50 µm. En particulier, on peut utiliser les fibres de polyamide commercialisées par les Etablissements P. Bonte sous le nom Polyamide 0.9 Dtex 3 mm, ayant un diamètre moyen de 6 µm, un titre d'environ 0.9 dtex et une longueur allant de 0,3 mm à 5 mm. On peut aussi utiliser les fibres de poly-p-phénylène téréphtalamide de diamètre moyen de 12 µm et de longueur d'environ 1,5 mm comme celles vendues sous le nom de Kevlar Floc par la société Du Pont Fibres ou bien encore les fibres de celluloses (ou de rayonne) ayant un diamètre moyen de 50 µm et une longueur allant de 0,5 mm à 6 mm comme celles vendues sous le nom de Natural rayon flock fiber RC1 BE ― N003 ― M04 par la société Claremont Flock. On peut également utiliser des fibres de polyéthylène comme celles vendues sous le nom de Shurt Stuff 13 099 F par la société Mini Fibers.

Les deuxièmes fibres peuvent êtres présentes dans la composition selon l'invention en une teneur allant de 0,01 à 10 % en poids, par rapport au poids total de la composition, de préférence de 0,1 % à 5 % en poids, et mieux de 0,3 % à 2 % en poids.

Avantageusement, les première et deuxième fibres peuvent être présentes dans la composition selon l'invention selon un rapport pondéral deuxième fibre / première fibre (fibrilles de cellulose) allant de 0,8 à 2,5, et mieux allant de 1 à 2.

Le milieu aqueux de la composition peut comprendre ou être constitué essentiellement d'eau, et éventuellement d'un solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄.

L'eau, et éventuellement le solvant organique miscible à l'eau, peut être présente, en une teneur allant de 1 % à 95 % en poids, par rapport au poids total de la composition, de préférence de 5 % à 80 % en poids, et mieux de 10 % à 60 % en poids.

La composition selon l'invention peut comprendre en outre au moins une cire.
Par "cire", on entend au sens de la présente invention, un composé gras lipophile, solide à température ambiante (25 °C) et pression atmosphérique (760 mm de Hg, soit 10⁵ Pa), à changement d'état solide/liquide réversible, ayant une température de fusion supérieure à 30 °C et mieux supérieure à 55 °C et pouvant aller jusqu'à 200 °C, notamment jusqu'à 120 °C.
En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER , avec une montée en température de 5 ou 10 °C par minute.

Les cires, au sens de l'invention, sont celles généralement utilisées dans les domaines cosmétique et dermatologique . On peut notamment citer la cire d'abeilles, la cire de lanoline, et les cires d'insectes de Chine; la cire de riz, la cire de Carnauba, la cire de Candellila, la cire d'Ouricury, la cire de fibres de liège, la cire de canne à sucre, la cire du Japon et la cire de sumac; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40°C et mieux à plus de 55°C.
On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée.
On peut encore citer les cires de silicone ou les cires fluorées.

Les cires présentes dans la composition peuvent être dispersées sous forme de particules dans un milieu aqueux. Ces particules peuvent avoir une taille moyenne allant de 50 nm à 10 µm, et de préférence de 50 nm à 3,5 µm.
En particulier, la cire peut être présente sous forme d'émulsion cires-dans-eau, les cires pouvant être sous forme de particules de taille moyenne allant de 1 µm à 10 µm, et de préférence de 1 µm à 3,5 µm.
Dans un autre mode de réalisation de la composition selon l'invention, la cire peut être présente sous forme de microdispersion de cire, la cire étant sous forme de particules dont la taille moyenne est inférieure à 1 µm, et va notamment de 50 nm à 500 nm. Des microdispersions de cires sont décrites dans les documents EP-A-557196, EP-A-1048282.

La cire peut également présenter une dureté allant de 0,05 MPa à 15 MPa, et de préférence allant de 6 MPa à 15 MPa . La dureté est déterminée par la mesure de la force en compression mesurée à 20 °C à l'aide du texturomètre vendu sous la dénomination TA-XT2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par la surface du cylindre du texturomètre en contact avec la cire.

La cire peut être présente dans la composition selon l'invention en une teneur allant de 0,1 % à 50 % en poids, par rapport au poids total de la composition, de préférence de 0,5 % à 30 % en poids, et mieux de 1 % à 20 % en poids.

La composition selon l'invention peut comprendre au moins une huile ou solvant organique volatile et/ou au moins une huile non volatile.

Par " huile ou solvant organique volatile", on entend au sens de l'invention tout milieu non aqueux susceptible de s'évaporer au contact de la peau en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 10⁻² à 300 mm de Hg (1,33 Pa à 40.000 Pa) et de préférence supérieure à 0,3 mm de Hg (30 Pa). Par "huile non volatile", on entend une huile restant sur la peau à température ambiante et pression atmosphérique au moins plusieurs heures et ayant notamment une pression de vapeur inférieure à 10⁻² mm de Hg (1,33 Pa).

Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C₈-C₁₆ le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité ≤ 8 centistokes (8 10⁻⁶ m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également utiliser des solvants volatils fluorés tels que le nonafluorométhoxybutane ou le perfluorométhylcyclopentane.

L'huile volatile peut être présente dans la composition selon l'invention en une teneur allant de 0,1 % à 98 % en poids, par rapport au poids total de la composition, de préférence de 1 % à 65 % en poids.

La composition peut également comprendre au moins une huile non volatile, et notamment choisie parmi les huiles hydrocarbonées et/ou siliconées et/ou fluorées non volatiles.
Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, le benzoate d'alcool en C₁₂ à C₁₅, le laurate d'hexyle, l'adipate de diisopropyle, l'isononanoate d'isononyle, le palmitate de 2-éthyl-hexyle, l'isostéarate d'isostéaryle, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'invention sont notamment des huiles fluorosiliconées, des polyéthers fluorés, des silicones fluorées telles que décrit dans le document EP-A-847752.

Les huiles non volatiles peuvent être présentes dans la composition selon l'invention en une teneur allant de 0,1 % à 80 % en poids, de préférence de 0,1 % à 50 % en poids, par rapport au poids total de la composition, et mieux de 0,1 % à 20 % en poids.

La composition selon l'invention peut contenir des agents tensioactifs émulsionnants présents notamment en une proportion allant de 2 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges.
- parmi les tensioactifs anioniques : les acides gras en C₁₆-C₃₀ neutralisés par les amines, l'ammoniaque ou les sels alcalins, et leurs mélanges.

On utilise de préférence des tensioactifs permettant l'obtention d'émulsion huile-dans-eau ou cire-dans-eau.

La composition selon l'invention peut comprendre au moins un polymère filmogène.

Le polymère filmogène peut être un polymère solubilisé ou dispersé sous forme de particules solides dans une phase aqueuse de la composition ou bien encore solubilisé ou dispersé sous forme de particules solides dans une phase grasse liquide. La composition peut comprendre un mélange de ces polymères. Lorsque le polymère filmogène se présente sous forme de particules solides, ces particules peuvent présenter une taille moyenne de particules allant de 5 nm à 600 nm, et de prféréence de 20 nm à 300 nm.

Le polymère filmogène peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Dans la présente demande, on entend par "polymère filmogène", un polymère apte à former à lui seul ou en présence d'un agent auxiliaire de filmification, un film continu et adhérent sur un support, notamment sur les matières kératiniques.

On utilise de préférence un polymère filmogène apte à former un film hydrophobe, c'est-à-dire un polymère dont le film a une solubilité dans l'eau à 25 °C inférieure à 1 % en poids.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).
Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₃₀, de préférence en C₁-C₂₀, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

Il est possible d'utiliser tout monomère connu de l'homme du métier entrant dans les catégories de monomères acryliques et vinyliques (y compris les monomères modifiés par une chaîne siliconée).

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les polyuréthanes-polyvinylpirrolidones, les polyester-polyuréthanes, les polyétherpolyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol.
Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.
On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ® par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de la composition selon l'invention, le polymère filmogène peut être présent sous la forme de particules en dispersion aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90®, NEOCRYL A-1070®, NEOCRYL A-1090®, NEOCRYL BT-62®, NEOCRYL A-1079®, NEOCRYL A-523® par la société AVECIA-NEORESINS, DOW LATEX 432® par la société DOW CHEMICAL, DAITOSOL 5000 AD® par la société DAITO KASEY KOGYO; ou ben encore les dispersions aqueuses de polyuréthane vendues sous les dénominations NEOREZ R-981®, NEOREZ R-974® par la société AVECIA-NEORESINS, les AVALURE UR-405®, AVALURE UR-410®, AVALURE UR-425 ®, AVALURE UR-450®, SANCURE 875®, SANCURE 861®, SANCURE 878®, SANCURE 2060® par la société GOODRICH, IMPRANIL 85® par la société BAYER, AQUAMERE H-1511® par la société HYDROMER.

Comme dispersion aqueuse de polymère filmogène, on peut également utiliser les dispersions de polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

Selon une deuxième variante de réalisation de la composition selon l'invention, le polymère filmogène peut être un polymère hydrosoluble et est donc présent dans la phase aqueuse de la composition sous forme solubilisée. Comme exemples de polymères filmogènes hydrosolubles, on peut citer
- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :
   . les gommes arabiques, la gomme de guar, les dérivés du xanthane, la gomme de karaya ;
   . les alginates et les carraghénanes ;
   . les glycoaminoglycanes, l'acide hyaluronique et ses dérivés ;
   . la résine shellac, la gomme de sandaraque, les dammars, les élémis, les copals ;
   . l'acide désoxyribonucléïque ;
   . les muccopolysaccharides tels que l'acide hyaluronique, les chondroïtines sulfate, et leurs mélanges.

Selon une autre variante de réalisation de la composition selon l'invention, le polymère filmogène peut être présent dans une phase grasse liquide comprenant des huiles ou solvants organiques tels que ceux décrits précédemment. Par "phase grasse liquide", on entend, au sens de l'invention, une phase grasse liquide à température ambiante (25°C) et pression atmosphérique (760 mm de Hg, soit 10⁵ Pa), composée d'un ou plusieurs corps gras liquides à température ambiante, appelés aussi huiles, généralement compatibles entre eux.
De préférence, la phase grasse liquide comprend une huile volatile, éventuellement en mélange avec une huile non volatile, les huiles pouvant être choisies parmi les huiles citées précédemment.

Selon un troisième mode de réalisation de la composition selon l'invention, le polymère filmogène peut être présent sous forme de particules, stabilisées en surface, dispersées dans la phase grasse liquide.

La dispersion de particules de polymère stabilisées en surface peut être fabriquée comme décrit dans le document EP-A-749747.

Les particules de polymère sont stabilisées en surface grâce à un stabilisant qui peut être un polymère séquencé, un polymère greffé, et/ou un polymère statistique, seul ou en mélange.

Des dispersions de polymère filmogène dans la phase grasse liquide, en présence d'agent stabilisants, sont notamment décrites dans les documents EP-A-749746, EP-A-923928, EP-A-930060 dont le contenu est incorporé à titre de référence dans la présente demande.

La taille des particules de polymères en dispersion soit dans la phase aqueuse, soit dans la phase grasse liquide, peut aller de 5 nm à 600 nm, et de préférence de 20 nm à 300 nm.

Selon un quatrième mode de réalisation de la composition selon l'invention, le polymère filmogène peut être solubilisé dans la phase grasse liquide, on dit alors que le polymère filmogène est un polymère liposoluble.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvlnyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 10 à 20 atomes de carbone.

De tels homopolymères liposolubles peuvent être choisis parmi le polystéarate de vinyle, le polystéarate de vinyle réticulé à l'aide de divinylbenzène, de diallyléther ou de phtalate de diallyle, le poly(méth)acrylate de stéaryle, le polylaurate de vinyle, le poly(méth)acrylate de lauryle, ces poly(méth)acrylates pouvant être réticulés à l'aide de diméthacrylate de l'éthylène glycol ou de tétraéthylène glycol.

Les copolymères et homopolymères liposolubles définis précédemment sont connus et notamment décrits dans la demande FR-A-2262303 ; ils peuvent, avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C₂-C₂₀, comme le polybutène, les alkylcelluloses avec un radical alkyle linéaire ou ramifié, saturé ou non en C₁ à C₈ comme l'éthylcellulose et la propylcellulose, les copolymères de la vinylpyrolidone (VP) et notamment les copolymères de la vinylpyrrolidone et d'alcène en C₂ à C₄₀ et mieux en C₃ à C₂₀. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

La composition selon l'invention peut comprendre un agent auxiliaire de filmification favorisant la formation d'un film avec le polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 50 % en poids, par rapport au poids total de la composition, de préférence allant de 0,01 % à 30 % en poids.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène.

La composition de l'invention peut comprendre, en outre, tout additif usuellement utilisé en cosmétique tels que les antioxydants, les charges, les conservateurs, les parfums, les neutralisants, les épaississants, les actifs cosmétiques ou dermatologiques comme par exemple des émollients, des hydratants, des vitamines, des filtres solaires, et leurs mélanges. Ces additifs peuvent être présents dans la composition en une teneur allant de 0,01 à 20 % du poids total de la composition et mieux de 0,01 à 10% (si présents).

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut être fabriquée par les procédés connus, généralement utilisés dans le domaine cosmétique ou dermatologique.

L'invention est illustrée plus en détail dans les exemples suivants.

### Exemple 1 :

On a préparé un mascara ayant la composition suivante :
- Cire de carnauba 2,6 g
- Cire d'abeille 3,3 g
- Cire de paraffine 10,3 g
- Huile de jojoba hydrogénée 0,2 g
- Huile de palme hydrogénée 0,2 g
- Amino-2 méthyl-2 propanediol-1,3 0,8 g
- Triéthanolamine 2,4 g
- Acide stéarique 6,6 g
- Polyméthacrylate de sodium (Darvan 7 de la société VANDERBILT) 0,25 g MA
- Hydroxyéthylcellulose réticulée par l'épichlorhydrine quaternisée par la triméthylamine (JR 400 de la société UNION CARBIDE) 0,1 g
- Copolymère acrylate d'éthyle/méthacrylate de méthyle (80/20) en dispersion aqueuse à 50 % MA (DAITOSOL 5000 AD de SAITO) 2,5 g MA
- Fibres de polyamide (3 mm de long et 0,9 Dtex de la société Paul Bonte) 1 g
- Microfibrilles de cellulose (CELLULON de la société MONSANTO KELCO) 0,5 g
- Oxyde de fer noir 7 g
- Conservateurs qs
- Eau qsp 100 g

Ce mascara s'applique facilement et adhère bien sur les cils pendant et après l'application.
Le maquillage obtenu confère un effet d'allongement des cils et la tenue des fibres de polyamide sur les cils est très bonne.

### Exemple 2 :

On a préparé un mascara ayant la composition suivante :
- Microfibrilles de cellulose (CELLULON de la société MONSANTO KELCO) 0,17 g
- Eau 9,3 g
- Fibres de polyamide (3 mm de long et 0,9 Dtex de la société Paul Bonte) 1 g
- Cire de carnauba 4,6 g
- Huile de jojoba hydrogénée 2,2 g
- Cire de paraffine 2,2 g
- Cire d'abeille 8,0 g
- Amidon de riz 0,8 g
- Bentonite 7 g
- Copolymère acétate de vinyle/stéarate d'allyle (65/35) (Mexomère PQ de CHIMEX) 2,0 g
- Polylaurate de vinyle (Mexomère PP de CHIMEX) 0,7 g
- Carbonate de propylène 2,3 g
- Pigments 4,6 g
- Conservateurs qs
- Isododécane qsp 100 g

Ce mascara waterproof allonge très bien les cils et les fibres de polyamide tiennent très bien sur les cils toute la journée.

## Revendications

1. Composition comprenant, dans un milieu aqueux physiologiquement acceptable, une première fibre choisie parmi les fibrilles de cellulose et une deuxième fibre différente de ladite première fibre.

2. Composition selon la revendication 1, **caractérisée en ce que** la première fibre a une plus grande longueur inférieure à 100 µm, de préférence inférieure ou égale à 50 µm.

3. Composition selon la revendication 1 ou 2, **caractérisée en ce que** la première fibre a une plus grande longueur allant de 5 à 40 µm.

4. Composition selon l'une quelconque des revendications précédentes, **caractérisée en ce que** la première fibre a un diamètre allant de 1 à 100 nm.

5. Composition selon l'une quelconque des revendications précédentes, **caractérisée par le fait que** la première fibre présente un rapport plus grande longueur/diamètre supérieur à 30.

6. Composition selon l'une quelconque des revendications précédentes, **caractérisée par le fait que** les fibrilles de cellulose comprennent de la cellulose carboxylée et/ou sont modifiées par des acides carboxyliques et/ou sont associées à un composé organique polyhydroxylé.

7. Composition selon l'une quelconque des revendications précédentes, **caractérisée en ce que** la première fibre est présente en une quantité allant de 0,01 % à 5 % en poids, par rapport au poids total de la composition, et de préférence allant de 0,1 % à 3 % en poids, et mieux allant de 0,5 % à 2 % en poids.

8. Composition selon l'une quelconque des revendications précédentes, **caractérisé par le fait que** la deuxième fibre a une longueur supérieure ou égale à 100 µm, de préférence allant de 100 µm à 10 mm, et mieux allant de 0,1 mm à 5 mm.

9. Composition selon l'une quelconque des revendications précédentes, **caractérisé par le fait que** la deuxième fibre est choisie parmi les fibres de soie, de coton, de laine, de lin, des fibres de cellulose, de polyamide, de rayonne, de viscose, d'acétate notamment d'acétate de rayonne, de poly-(p-phénylène-téréphtalamide), de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène, de collagène insoluble, de polyesters, de polychlorure de vinyle ou de vinylidène, d'alcool polyvinylique, de polyacrylonitrile, de chitosane, de polyuréthane, de polyéthylène phtalate, des fibres de mélanges de polymères.

10. Composition selon l'une quelconque des revendications précédentes, **caractérisé par le fait que** la deuxième fibre est une fibre d'origine synthétique.

11. Composition selon l'une quelconque des revendications précédentes, **caractérisé par le fait que** la deuxième fibre est choisie parmi les fibres de polyamide, de poly-(p-phénylène-téréphtalamide), de cellulose ou de polyéthylène.

12. Composition selon l'une quelconque des revendications précédentes, **caractérisée en ce que** la deuxième fibre a une longueur L et un diamètre D tel que L/D est choisi dans la gamme allant de 3,5 à 2 500, de préférence de 5 à 500 et mieux de 5 à 150.

13. Composition selon l'une quelconque des revendications précédentes, **caractérisée par le fait que** la deuxième fibre a un titre choisi dans la gamme allant de 0,15 à 30 deniers et mieux de 0,18 à 18 deniers.

14. Composition selon l'une quelconque des revendications précédentes, **caractérisé par le fait que** la deuxième fibre a une section comprise dans un cercle de diamètre allant de 2 nm à 500 µm.

15. Composition selon l'une quelconque des revendications précédentes, **caractérisé par le fait que** la deuxième fibre est présente en une teneur allant de 0,01 % à 10 % en poids, par rapport au poids total de la composition, de préférence de 0,3 % à 2 % en poids.

16. Composition selon l'une quelconque des revendications précédentes, **caractérisée par le fait qu'**elle comprend une cire.

17. Composition selon l'une quelconque des revendications précédentes, **caractérisée par le fait qu'**elle contient au moins une cire ayant une température de fusion supérieure à 30°C et allant jusqu'à 120 °C.

18. Composition selon l'une quelconque des revendications précédentes, **caractérisée par le fait qu'**elle contient une cire choisie dans le groupe formé par la cire d'abeilles, la cire de lanoline, les cires d'insectes de Chine, la cire de riz, la cire de Carnauba, la cire de Candellila, la cire d'Ouricury, la cire de fibres de liège, la cire de canne à sucre, la cire du Japon et la cire de sumac, la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40°C, les cires obtenues par hydrogénation catalytique d'huiles animales ou végétales ayant des chaînes grasses, linéaires ou ramifiées, en C8-C32, les cires de silicone, les cires fluorées.

19. Composition selon l'une quelconque des revendications précédentes, **caractérisée par le fait qu'**elle comprend une cire ayant une dureté allant de 0,05 MPa à 15 MPa.

20. Composition selon l'une quelconque des revendications 16 à 19, **caractérisée par le fait que** la cire est présente en une teneur allant de 0,1 % à 50 % en poids, par rapport au poids total de la composition, de préférence de 0,5 % à 30 % en poids, et mieux de 1 % à 20 % en poids.

21. Composition selon l'une quelconque des revendications précédentes, **caractérisée par le fait qu'**elle contient une huile ou un solvant organique volatile.

22. Composition selon la revendication 21, **caractérisée par le fait que** l'huile volatile est une choisie parmi les huiles volatiles hydrocarbonées ayant de 8 à 16 atomes de carbone.

23. Composition selon la revendication 21 ou 22, **caractérisée par le fait que** l'huile volatile est présent en une teneur allant de 0,1 % à 98 % en poids, par rapport au poids total de la composition, et de préférence allant de 1 % à 65 % en poids.

24. Composition selon l'une des revendications précédentes, **caractérisée par le fait qu'**elle comprend une huile non volatile.

25. Composition selon l'une des revendications précédentes, **caractérisée par le fait qu'**elle contient, en outre, au moins une huile non volatile choisie parmi les huiles hydrocarbonées d'origine minérale, végétale ou synthétique, les esters ou éthers de synthèse, les huiles de silicone et leurs mélanges.

26. Composition selon l'une quelconque des revendications précédentes, **caractérisée par le fait que** le milieu aqueux comprend de l'eau et éventuellement un solvant organique miscible à l'eau.

27. Composition selon l'une quelconque des revendications précédentes, **caractérisée par le fait qu'**elle comprend de l'eau en une teneur allant de 1 à 95 % en poids, par rapport au poids total de la composition, de préférence allant de 5 % à 80 % en poids, et mieux allant de 10 % à 60 % en poids.

28. Composition selon l'une quelconque des revendications précédentes, **caractérisée par le fait qu'**elle comprend un polymère filmogène.

29. Composition selon la revendication 28, **caractérisée par le fait que** le polymère filmogène est choisi dans le groupe formé par les polymères vinyliques, les polyuréthanes, les polyesters, les polyamides, les polyurées, les polymères cellulosiques.

30. Composition selon la revendication 28 ou 29, **caractérisée par le fait que** le polymère filmogène est solubilisé dans une phase aqueuse ou sous forme de particules en dispersion aqueuse.

31. Composition selon l'une quelconque des revendications 28 à 30, **caractérisée par le fait que** le polymère filmogène est solubilisé ou dispersé sous forme de particules stabilisées en surface dans une phase grasse liquide.

32. Composition selon l'une quelconque des revendications 28 à 31, **caractérisée par le fait que** le polymère filmogène est présent en une teneur allant de 0,1 % à 60 % en poids, par rapport au poids total de la composition, de préférence de 0,5 % à 40 % en poids, et mieux de 1 % à 30 % en poids.

33. Composition selon l'une des revendications précédentes, **caractérisée par le fait qu'**elle contient, en outre, au moins une matière colorante.

34. Composition selon la revendication 33, **caractérisée par le fait que** la matière colorante est choisie parmi les pigments, les nacres, les colorants liposolubles, les colorants hydrosolubles.

35. Composition selon la revendication 33 ou 34, **caractérisée par le fait que** la matière colorante est présente à raison de 0,01 à 50 % du poids total de la composition, de préférence allant de 0,01 % à 30 % en poids.

36. Composition selon l'une des revendications précédentes, **caractérisée par le fait qu'**elle constitue une composition de soin ou de maquillage des matières kératiniques.

37. Composition selon l'une des revendications précédentes, **caractérisée par le fait qu'**elle contient au moins un additif choisi parmi les antioxydants, les charges, les conservateurs, les parfums, les neutralisants, les épaississants, les actifs cosmétiques ou dermatologiques.

38. Composition selon l'une des revendications précédentes, **caractérisée par le fait qu'**elle se présente sous forme de mascara, d'eye-liner, de produit pour les sourcils, de produit pour les lèvres, de fard à joues ou à paupières, de fond de teint, de produit de maquillage du corps, de produit anti-cernes, de vernis à ongles, de produit de soin de la peau, de produit pour les cheveux.

39. Mascara comprenant une composition selon l'une quelconque des revendications 1 à 37.

40. Procédé cosmétique de maquillage ou de soin des matières kératiniques des êtres humains, comprenant l'application sur les matières kératiniques d'une composition cosmétique conforme à l'une des revendications 1 à 39.

41. Utilisation d'une composition selon l'une quelconque des revendications 1 à 38 pour obtenir une bonne tenue des fibres sur les matières kératiniques.

42. Utilisation de fibrilles de cellulose dans une composition, notamment un mascara, comprenant dans un milieu aqueux cosmétiquement acceptable, des fibres, différentes desdites microfibrilles de cellulose, pour obtenir un bonne tenue des fibres sur les matières kératiniques, notamment les cils.
